# EUROPEAN PATENT SPECIFICATION

(11) **EP 1 347 796 B1**
(45) Date of publication and mention of the grant of the patent: **17.08.2005**
(21) Application number: 02734865.5
(22) Date of filing: 04.01.2002
(51) Int. Cl.: A61M 5/32

(54) **IMPROVED HYPODERMIC NEEDLE AND FLUID INJECTION DEVICE**
VERBESSERTE INJEKTIONSNADEL UND FLÜSSIGKEITSINJEKTIONSVORRICHTUNG
AIGUILLE HYPODERMIQUE AMELIOREE ET DISPOSITIF D'INJECTION DE FLUIDE

(30) Priority: 05.01.2001 GB 0100247
(43) Date of publication of application: 01.10.2003
(73) Proprietor: University of Dundee, Dundee DD1 4HN (GB)
(72) Inventor: CUSCHIERI, Alfred, St. Andrews, Fife K19 9TY (GB); FRANK, Timothy Graham, Fife DD6 8NG (GB); XU, Wei, School of Mech. & Materials Engineering, Guildford, Surrey GU2 5XH (GB)
(74) Representative: Chapman, Paul Gilmour
(86) International application number: PCT/GB2002/000048
(87) International publication number: WO 2002/058768

(56) References cited:
- WO-A-00/40281
- WO-A-00/54660
- GB-A- 2 327 614
- US-A- 5 354 279

## Description

The present invention relates to an improved hypodermic needle and fluid injection device. In particular, but not exclusively, the present invention relates to a hypodermic needle having a first hollow needle having movably secured therein one or more further hollow needles and a fluid injection device including such a hypodermic needle. See for example GB-A-2 327 614.

There are several medical situations where it is desired to deliver substances by injection to a relatively large volume of tissue. If a substance is delivered by a single point injection, the problems that may arise are (a) the substance cannot spread throughout the volume in sufficient time; (b) too much dilution may occur during the spreading; (c) the distribution of the substance within the volume may be very inhomogeneous; and (d) unwanted spreading to regions away from the target volume may occur. One solution to this problem is to give smaller injections at several sites within the target volume. This approach has at least three disadvantages: (a) multiple needle stab wounds are created; (b) accuracy of placement is limited; and (c) the time for the procedure is increased.

A particular example of these difficulties arises in the treatment of liver cancers. Cancers within the liver can be killed with large doses of alcohol. One surgical approach is to expose the liver using open or laparoscopic surgery and inject the tumour at many sites using the same needle. One severe problem of this approach is that considerable bleeding results from the many stab wounds and, more seriously, this bleeding can carry tumour cells into the peritoneal cavity.

It is amongst the objects of the present invention to obviate or mitigate at least one of the foregoing disadvantages.

According to a first aspect of the present invention, there is provided a fluid injection device including a hypodermic needle comprising a first, hollow needle adapted to have movably secured therein two or more further, hollow needles, /each said further needle and part of the hypodermic needle being movable relative to one another between a stressed position of /each further needle, in which /each said further needle is substantially parallel to the first needle, and an unstressed position of /each further needle, in which the free end of /each said further needle lies beyond the axial and/or radial terminus of the first needle, the device further comprising driving means for driving fluid from the two or more further hollow needles of the hypodermic needle, when in the unstressed position, at a substantially constant fluid flow rate.

The hypodermic needle includes two or more further needles. In the unstressed position, /each said further needles may protrude beyond both the axial and radial termini of the first needle.

A particular advantage is the ability of each further needle to spread outwardly from the first hollow needle, thereby permitting injection into a large volume of tissue from a single puncture wound (in the case of plural, further needles); and/or allowing injection to one or more sites remote from the terminus of the first needle. A further advantage is that this arrangement, including the driving means, may assist in allowing the fluid to be discharged and injected into the tissue at a substantially constant flow rate from /each further needle, to achieve a desired distribution of injected fluid. Furthermore, the creation of a single wound minimises bleeding; can for example reduce the above-noted risk of transfer of cancer cells; and speeds the operation.

The at least two further hollow needles are preferably movable relative to the first hollow needle, and the at least two further hollow needles may extend through a single opening in an open lower end of the first hollow needle, the lower end serving for puncturing tissue.

The drive means may be automatic, that is, automatically rather than manually actuated.

According to a second aspect of the present invention, there is provided a hypodermic needle comprising:
a first tapered hollow needle portion having first and second parts, the first part being of greater diameter than the second part;
two or more further hollow needles; and
securing means for location in the first part of the first tapered hollow needle portion, for receiving and movably securing the two or more further hollow needles therein;
wherein the two or more further hollow needles are more closely packed in the second part of the first hollow needle portion than in the first part.

According to a third aspect of the present invention, there is provided a fluid injection device including:
a hypodermic needle comprising a first tapered hollow needle portion having first and second parts, the first part being of greater diameter than the second part; two or more further hollow needles; and securing means for location in the first part of the first tapered hollow needle portion, for receiving and movably securing the two or more further hollow needles therein, wherein the two or more further hollow needles are more closely packed in the second part of the first hollow needle portion than in the first part; and
driving means for driving fluid from the two or more further hollow needles of the hypodermic needle, at a substantially constant fluid flow rate.

Preferably, each further needle and part of the hypodermic needle are moveable relative to one another between a stressed position of each further needle, in which each said further needle is substantially parallel to the first needle, and an unstressed position of each further needle, in which the free end of each said further needle lies beyond the axial and/or radial terminus of the first needle.

The first and second parts of the hypodermic needle may comprise generally tubular members, which are coupled together to form the first tapered hollow needle portion. The first tapered hollow needle portion may further comprise a coupling for coupling the first and second needle portion parts together. Conveniently, the first needle portion part is of an internal diameter greater than an external diameter of the second needle portion part, and the coupling comprises a tube adapter for sealingly coupling the first and second needle portion parts together.

The tube adapter may advantageously both seal the first needle portion part to the second needle portion part and provide a smooth transition surface from the first needle portion part to the second needle portion part. This may advantageously assist in insertion of the first tapered hollow needle portion into subject tissue.

Conveniently, the tube adapter comprises a generally tubular member located between an outer surface of the second needle portion part and an inner surface of the first needle portion part, and includes a tapered end for providing a smooth transition from an outer surface of the first needle portion part to the outer surface of the second needle portion part. The tube adapter may include a radial shoulder defining an abutment surface for abutting an end of the first needle portion part.

Alternatively, the first tapered hollow needle portion may comprises a single, substantially one-piece needle portion, which tapers from the first diameter of the first part to the second diameter of the second part. It will be understood that, generally speaking, both the internal and external diameters of the first needle portion part are greater than the respective internal and external diameters of the second needle portion part.

The hypodermic needle may further comprise a generally tubular needle guide adapted to be located within the first tapered hollow needle portion. The needle guide may be disposed in the first needle portion part and may taper internally towards an end thereof disposed, in use, nearest the second needle portion part, to define a tapered passage. Walls of the tapered passage may act as a guide for the two or more further hollow needles. In this fashion, the two or more further hollow needles, which are located, in use, within the first tapered hollow needle portion, may be more closely packed in the second needle portion part than in the first needle portion part. This may be achieved by threading the two or more further hollow needles through the needle guide. The tubular needle guide may alternatively comprise first and second parts which may be axially separated along the further needles. Such structure may be provided where there are a plurality of further needles which are axially staggered. One or both of the first and second parts may taper internally. The needle guide may assist in assembly of the hypodermic needle by retaining the further needles in the stressed position during location in the needle first tapered hollow needle portion.

The securing means may comprise a needle manifold for mounting the two or more further hollow needles in the first needle portion part. The needle manifold may comprise a generally cylindrical body having passages for receiving the two or more further hollow needles. The needle manifold may seal the further hollow needles in the first tapered hollow needle portion, for directing injected fluid therethrough.

The hypodermic needle may include indexing means for allowing selective supply through one of the two or more further needles. Where more than two further needles are provided, the indexing means may allow supply through selected ones of the further needles. The indexing means may include a feed manifold, which may be axially movable between a position where it allows supply to all of the further needles and a position where it allows supply to one or selected ones of the further needles. The feed manifold may be located in the first needle portion part and may be rotatable and axially movable.

One or more parts of the hypodermic needle, for example, the two or more further hollow needles, the coupling, the needle guide and/or the needle manifold may be removable to allow the parts to be sterilised for reuse. This further advantageously allows the one or more further needles to be sharpened, if required. Alternatively, the hypodermic needle may be disposable. For a wholly re-useable device, parts of the needles other than the further hollow needles may be of stainless steel; the two or more further hollow needles are most suitably of a shape memory alloy (SMA). Use of plastics for parts other than the two or more further hollow needles may allow the device to be disposable and to be constructed more cheaply. In this fashion, the SMA needles may be disposable or separately retained and sterilised, while plastic parts may be disposable. Construction in SMA, in combination with plastics, favours use of the hypodermic needle during magnetic resonance imaging (MRI) procedures, due to the good artefact produced by carefully treated SMA.

Preferably, the hypodermic needle comprises seven further needles. In an alternative embodiment, it may comprise four. Preferably also, each further needle is of the same length and internal diameter, allowing the fluid to be discharged from each further needle at substantially uniform fluid flow rates. At least one of the further needles may be axially staggered with respect to the one or more other further needles, such that an inlet of the at least one further staggered needle is axially spaced from a/each inlet of the one or more further needles. An advantage of such an arrangement comprising two or more further needles of the same length and internal diameter is that this may assist in allowing a substantially constant, substantially uniform flow of fluid from the further needles, to achieve a uniform distribution of fluid in the injected tissue. This may be of particular importance in carrying out a successful treatment on the tissue. It will be understood that it is the provision of two or more further needles of the same length and internal diameter which is the primary factor in allowing a substantially uniform flow of fluid from the further needles, and that the driving means allows a substantially constant driving force to be exerted on the fluid, to provide a constant flow. This may allow automatic fluid discharge, avoiding problems of, for example, manual discharge by plunger depression as in conventional hypodermic needles.

The driving means may further comprise an injection chamber for storing a fluid to be driven from the two or more further needles. The driving means may comprise one injection chamber coupled to each further needle, to allow simultaneous driving of the fluid from each further needle. Alternatively, the driving means may comprise two or more injection chambers each coupled to a respective one of the two or more further needles, each injection chamber being adapted to allow fluid to be simultaneously driven from the further needles. The/each injection chamber may comprise an injection barrel or cylinder, and the driving means may further include a piston adapted to be received in the injection barrel or cylinder. The piston may be hydraulically, pneumatically, electrically, mechanically or electro-mechanically activated to discharge the fluid from the injection chamber. Alternatively, the driving means may include a pump or the like coupled to the injection chamber. The driving means may be activated in response to a control command issued by an operator of the device. The/each injection chamber may be coupled to the hypodermic needle by a supply tube, or the/each injection chamber may be integral with the hypodermic needle.

Preferably, each said further needle is moveable relative to the remainder of the hypodermic needle and lies within the first needle when occupying its stressed position and protrudes from the first needle when occupying its unstressed position.

Preferably, each said further needle is generally straight when occupying its stressed position and curved when occupying its unstressed position, thereby permitting the free end of each further needle to project beyond the axial and radial terminus of the first needle when each further needle occupies its unstressed position.

This advantageously allows the further needles to be stored within the first needle, for example, during the puncturing operation and when the apparatus is being transported.

In particularly preferred embodiments, each said further needle includes a circular arc when occupying its unstressed position. The or each said further needle may also include an arc in the range of curvature of 60° to 110°, when occupying its unstressed position. The arc may in alternative embodiments lie outside this range of curvature, or may be of infinitely large radius, thus one or more of the further needles may optionally include a straight portion. In particular, where seven further hollow needles are provided, at least one of said needles may be straight in both positions, and may be located centrally of said needles. The foregoing features advantageously assist the or each further needle to advance through, for example, the tissue without causing collateral damage thereto.

Preferably each said further needle is formed of or from a superelastic shape memory alloy (SMA), in particular a heat treated alloy comprising approximately 55.5% Ni and 44.5% Ti by weight. Preferably the cold drawn alloy is heat treated at approximately 500°C for about 15 minutes whilst secured on a former, and the alloy is then quenched in cold water, thereby conferring the preferred shape on the further needles when occupying the unstressed position. These features permit the ready transition from a substantially straight to a substantially curved configuration on advancement of the further needles from within the first, hollow needle. The superelastic shape memory alloy is particularly suitable for embodiments of a said further needle the ratio of whose unstressed radius of curvature to its diameter lies typically in the range of 25:1 to 5.1.

An SMA is preferred to spring stainless steel for reasons including that it has a recoverable strain some ten times greater than steel. Thus for a given curve the SMA tube can have up to ten times the diameter of a steel tube; conversely, for a given tube diameter, SMA can have a curve radius down to a tenth of that for steel. Preferably, the first hollow needle is of a NiTi SMA or plastics material suitable for use with a Magnetic Resonance Imaging (MRI) guidance system. NiTi SMA and plastics materials have MRI artefacts which are sufficient for use with an MRI system, such that operation and guidance of the hypodermic needle may be controlled using the MRI system.

Nonetheless, it may be suitable to use steel or other non-superelastic materials for the further needles, eg. in embodiments in which the needle radius of curvature lies outside the range specified above, and/or where an MRI guidance system is not employed. The free end of one or more of the further needles may be mutually axially spaced from the free end of the/one of the other further needles.

Preferably, the free end of the or each said further needle whose free end is disposed mutually axially spaced from the free end of the one of the other further needles is spaced axially and radially from the free ends of the/one of the other said further needles when the further needles occupy their extended positions.

Conveniently, the or each said further needle communicates with the interior of a hollow, elongate tube movably lying within the first said needle, the end of the hollow tube remote from the said terminus of the first needle engaging or including a moveable actuator member for advancing and retracting the or each said further needle relative to the first needle. This may provide a convenient and simple means of moving the or each further needle between its stressed and unstressed positions.

In an alternative embodiment, each of the further needles may be independently movably secured within the first hollow needle, such that each further needle is independently movable relative to the one or more further needles and to the first hollow needle. This may advantageously allow each further needle to be independently moved between the stressed and unstressed positions by an operator, to locate each further needle in a desired location. This may be advantageously assisted by monitoring the location of each further needle during movement using the MRI guidance system. Each further needle may therefore be movable between the stressed and unstressed positions, and may be disposed in a position intermediate said stressed and unstressed positions for injection of the fluid.

According to a fourth aspect of the present invention, there is provided a hypodermic needle comprising a first, hollow needle having movably secured therein two or more further, hollow needles, each said further needle and part of the hypodermic needle being movable relative to one another between a stressed position of each further needle, in which each said further needle is substantially parallel to the first needle, and an unstressed position of each further needle, in which the free end of each said further needle lies beyond the axial and/or radial terminus of the first needle; and
wherein each further needle is of substantially the same length and internal diameter, for discharging a fluid from each further needle at a substantially uniform fluid flow rate, and wherein at least one of the further needles is axially staggered with respect to the one or more other further needles, such that an inlet of the at least one further staggered needle is axially spaced from a/each inlet of the one or more other further needles.

Preferably, the hypodermic needle forms part of a fluid injection device including driving means for driving fluid from the two or more further hollow needles at a substantially constant fluid flow rate. The driving means may include an injection chamber for storing a fluid to be discharged from the two or more further needles.

According to a fifth aspect of the present invention, there is provided a hypodermic needle comprising a first, hollow needle adapted to have movably secured therein a further hollow needle, the further needle and the first hollow needle being movable relative to one another between a stressed position of the further needle, in which the further needle is substantially parallel to the first needle, and an unstressed position of the further needle, in which the free end of the further needle lies beyond the axial and radial terminus of the first needle, the further needle being rotatable with respect to the first needle, for locating the free end of the further needle in a desired rotational orientation with respect to the first needle.

There follows a description of embodiments of the present invention, by way of example only, with reference to the accompanying drawings, in which:
Fig 1A is a partial longitudinal sectional side view of a hypodermic needle in accordance with an embodiment of the present invention, with further needles of the hypodermic needle shown in a retracted, stressed position;
Fig 1B is a cross-sectional view of the hypodermic needle shown in Fig 1A taken along line A-A of Fig 1A;
Fig 2 is a partial view of the hypodermic needle of Fig 1A, shown with the further needles in an extended, unstressed position;
Figs 3A and 3B are views of a preferred embodiment of a hypodermic needle similar to that shown in Fig 1A, but including seven further needles, coupled to a support tube and hypodermic syringe connector and shown in retracted, stressed and extended, unstressed positions of the further needles, respectively;
Fig 4 is a longitudinal cross-sectional view of the hypodermic needle shown in Figs 3A and 3B, shown in more detail;
Fig 5A is an enlarged view of an end of the hypodermic needle shown in Fig 4;
Fig 5B is a cross-sectional view of the hypodermic needle shown in Fig 5A, taken along line A-A of Fig 5A; and
Fig 6 is an enlarged partial view of the hypodermic needle of Fig 3B;
Fig 7 is a longitudinal cross-sectional view of part of a hypodermic needle in accordance with an alternative embodiment of the present invention, shown during assembly;
Figs 8A and 8B are views of the assembly of Fig 7 shown assembled and with further needles of the hypodermic needle in retracted, stressed positions;
Fig 8C is a view of the hypodermic needle of Fig 7, similar to the view of Fig 8B but showing the further needles in extended, unstressed positions, for discharging fluid through all of the further needles;
Fig 8D is an enlarged view of the hypodermic needle in the position of Fig 8C;
Fig 8E is a view of the hypodermic needle of Fig 7, in a position similar to that of Fig 8C, but with the hypodermic needle arranged to discharge fluid through a selected one of the further needles; and
Fig 8F is an enlarged view of the hypodermic needle of Fig 7 shown in the position of Fig 8E.

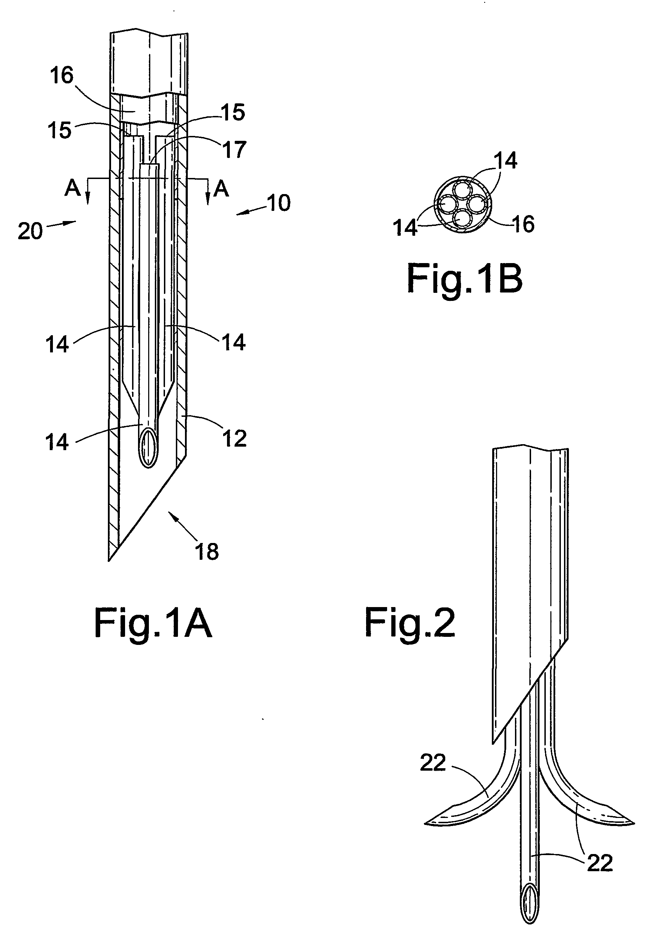

Referring now to Fig 1A of the drawings, there is shown a partial longitudinal sectional side view of a hypodermic needle 10, in accordance with an embodiment of the present invention. The hypodermic needle 10 comprises a first hollow outer needle 12, with four further, hollow needles 14 (three of which are shown in Fig 1A) movably secured within the first outer needle 12 by an inner supply tube 16.

The first outer needle 12 is of an outer diameter of approximately 2.4 mm, and an inner diameter of approximately 1.9 mm, and includes a free end 18 which is bevelled to form a sharp end for puncturing tissue to be treated, such as the liver and surrounding tissue of a patient. Each of the further hollow needles 14 have an outer diameter of approximately 0.63 mm and an inner diameter of approximately 0.42 mm, and are silver soldered to the inner supply tube 16 at an end 20 thereof. Each of the further hollow needles 14 are therefore sealed to the end 20 of the inner supply tube 16, for allowing discharge of a treatment fluid through the needles 14, as will be described in more detail below. Fig 1B is a cross-sectional view of the hypodermic needle 10 shown in Fig 1A, taken along line A-A, and with the first outer needle 12 not shown, for clarity. It will be seen that each of the further needles 14 are a tight fit within the inner supply tube 16, to provide a good seal when soldered.

Each of the further hollow needles 14 are constructed from a Shape Memory Alloy (SMA) comprising a 55.5% Ni - 44.5% Ti (by weight) NiTi alloy, and are shown in a retracted, stressed position in Fig 1A, where each of the further needles 14 are disposed entirely within and substantially parallel to the first outer needle 12. The inner supply tube 16 is movable axially with respect to the first outer needle 12, to deploy the further needles 14 from the retracted, stressed position shown in Fig 1A, to the extended, unstressed position shown in Fig 2. As will be described in more detail below, the NiTi alloy needles 14 exhibit superelastic properties, and are manufactured such that the natural, unstressed shape of each further needle 14 is as shown in Fig 2. Thus when the inner supply tube 16 is moved axially downwardly within the first outer needle 12, the further needles 14 are deployed from the free end 18 of the first outer needle 12, and assume the extended, unstressed position shown in Fig 2.

Each of the further hollow needles 14 are of the same length and internal diameter, as shown in Figs 1A and 1B. This ensures that when a treatment fluid is discharged from the free ends 22 of each further hollow needle 14, the fluid flows from each further needle 14 at a uniform fluid flow rate. This is important to ensure that a uniform dispersion of the treatment fluid within the tissue to be treated is obtained. Furthermore, the further needles 14 are axially staggered, such that the free ends 22, when the needles 14 are moved to the unstressed position, are disposed spaced around the target tissue, as shown in Fig 2. This results in a good distribution of the injected fluid over a target tissue volume. Specifically, inlets 15 of a first pair of the needles 14 are axially spaced from inlets 17 of a second pair of the needles 14 (only one of which is shown).

The inventors have observed that there is a poor distribution of treatment fluid within the tissue to be treated in prior art devices. The inventors ascertained that this was due to a non-uniform dispersal of the treatment fluid over the target tissue volume. It has been ascertained by the inventors that this is caused by using further needles of non-uniform length and/or internal diameter, which results in varying fluid flow rates of injected fluid from the needle free ends, a problem which is advantageously solved by the present invention.

The inner supply tube 16 and the first outer needle 12 are themselves constructed from an NiTi SMA, or alternatively from a plastics material, both of which have good Magnetic Resonance Imaging (MRI) artefacts, enabling the hypodermic needle 10 to be utilised with an MRI guidance system, as will be described in more detail below.

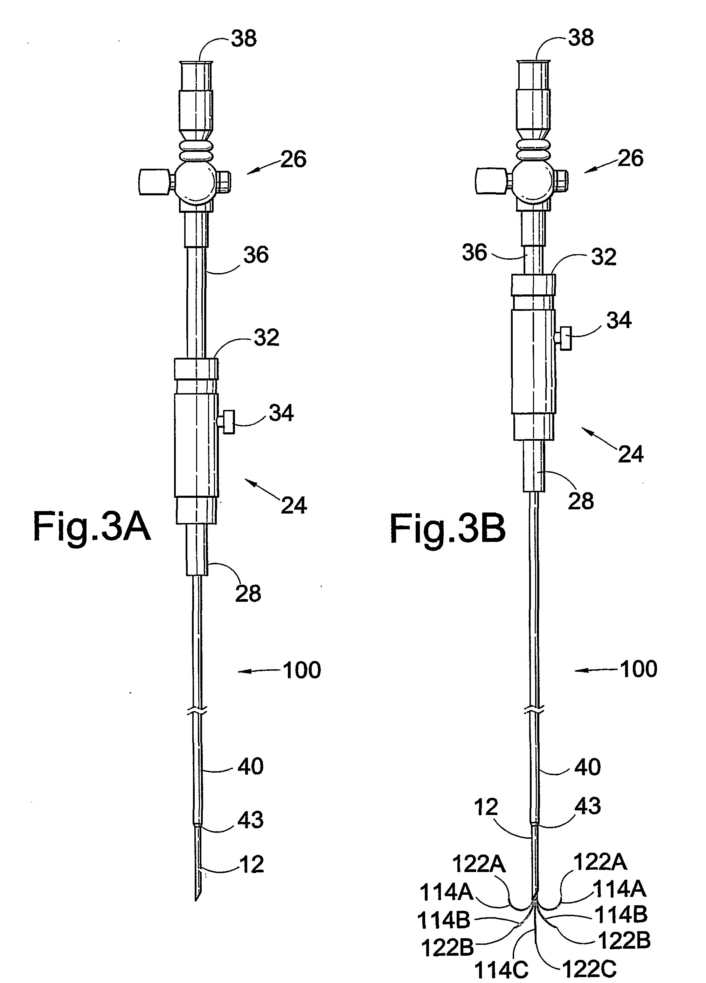

Referring now to Figs 3A and 3B, there are shown views of a preferred embodiment of a hypodermic needle 100, similar to the needle 10 of Figs 1A to 2. Like components of the needle 100 with the needle 10 share the same reference numerals incremented by 100. The hypodermic needle 100 is shown coupled to a support tube 24 and a standard hypodermic syringe connector 26. It will be understood that the hypodermic needle 10 may equally be coupled to such a support tube 24 and syringe connector 26. It will further understood that, in the following description, reference is made to use of the hypodermic needle 100, however, this applies equally to the hypodermic needle 10.

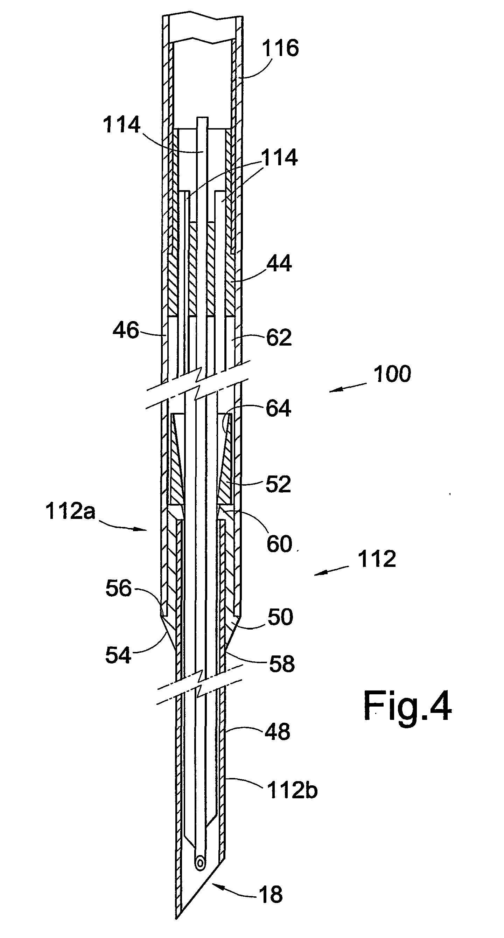

In Figs 3A and 3B, the hypodermic needle 100 is shown in retracted, stressed and extended, unstressed positions of the further needles, respectively. The structure of the hypodermic needle 100 will be described in more detail with reference to Figs 4 to 6 below. However, it will be noted from Fig 3B that the needle 100 includes seven further needles, generally designated 114. As shown, in the extended, unstressed position of Figure 3B, a first set of three further needles 114A (two shown) have a small radius of curvature and radiate symmetrically. Further, a second set of three needles 114B (two shown) have a larger radius of curvature and radiate symmetrically, but are axially rotated by 30° relative to the first set of needles 114A. A seventh, single further needle 114C is straight and lies on a main axis of the needle 100. Each needle has a respective free end 122A, 122B or 122C, as appropriate.

The further hollow needles 114 (and indeed the further hollow needles 14 of the hypodermic needle 10) are of internal diameters which provide a relatively large resistance to flow of fluid therethrough. This ensures that the resistance to fluid flow in the target tissue is overcome, ensuring uniform distribution of fluid in the target tissue. This is because the resistance to flow through the hollow needles is greater than that anticipated in typical target tissues. This also avoids any problems due to variations in flow resistances within the target tissue area.

Turning now to Figs 4 to 6, the hypodermic needle 100 is shown in more detail. The needle 100 comprises a first generally tapered hollow needle portion 112, seven further hollow needles 114, as described above, and securing means in the form of a needle manifold 44, for receiving and movably securing the needles 114 in the first needle portion 112. The needle portion 112 comprises a first part 46 and a second part 48, the first part 46 being of greater diameter than the second part 48. A coupling in the form of a tube adapter 50 couples the first needle portion part 46 to the second needle portion part 48, and a generally tubular needle guide 52 is provided in the first needle portion part 46.

Each of the further needles 114 are mounted in the needle manifold 44 in respective bores extending therethrough, which seal the further needles 114, such that fluid to be injected by the hypodermic needle 100 is directed through the further needles 114. An inner supply tube 116 is threadedly secured to the needle manifold 44, both for supplying fluid to be injected, and for moving the needles between retracted and extended positions, in a similar fashion to the needle 10 described above.

The tube adapter 50 includes a lower portion 54 which defines a shoulder 56 which receives and abuts an outer supply tube 112a of the first needle portion part 46. Furthermore, the tube adapter 50 defines a lower annular edge 58 and is closely engaged on a tube 112b of the second needle portion part 48. Thus the first and second needle portion parts 46 and 48 are coupled together and sealed to prevent fluid egress. Furthermore, the lower portion 54 of the adapter 50 is tapered, to define a smooth transition surface from the first needle portion part 46 to the second needle portion part 48, to assist in puncture and insertion of the needle 100.

The adapter 50 also includes an upper radial shoulder 60 which abuts the needle guide 52. The needle guide 52 is provided in a chamber 62 of the first needle portion part 46, and the further needles 114 extend from the first needle portion part 46, through the needle guide 52 to the second needle portion part 48. The needle guide 52 tapers internally towards an end disposed nearest the second needle portion part 48, and a wall 64 acts as a guide for the needles 122.

It will be noted that the further needles 114 are more closely packed in the first needle portion part 46 than in the second needle portion part 48. This is necessary because each of the needles 114 must be sealed in the needle manifold 44 to direct fluid through the needles. However, in providing seven further needles 114, the external diameter of the needle portion part 46 is relatively large. This would cause a relatively high trauma on insertion into a patient. Therefore, the needle guide 52 and the provision of the second needle portion part 48 of smaller diameter allows the needles 114 to be more closely packed as shown in Fig 4. This reduces likely trauma inflected on a patient, and advantageously allows the provision of multiple needles to achieve a good distribution of injected fluid.

Turning again to Figs 3A and 3B, it will be noted that the hypodermic needle 100 includes an upper collar 28, which is externally threaded for engaging the support tube 24. The support tube 24 includes an axial bore (not shown), the end of which adjacent the upper collar 28 is internally threaded for receiving and engaging the external threads of the upper collar 28, and to seal the hypodermic needle 100 to the support tube 24. An upper end 30 of the support tube 24 is externally threaded, for receiving and engaging an internally threaded collar 32 on the hypodermic syringe connector 26. A spring-loaded release button 34 is provided extending through a wall of the support tube 24, for engaging a coupling tube 36 of the syringe connector 26, by which the syringe connector 26 is coupled to the inner supply tube 116 of the hypodermic needle 100. This is achieved by providing an internally threaded collar (not shown) on a lower end of the coupling tube 36, which receives and engages the inner supply tube 116 via external threads thereon. The coupling tube 36 includes two circumferentially extending grooves, spaced axially along the coupling tube 36. These grooves correspond to the location of the syringe connector 26 when the further needles 114 of the hypodermic needle 10 are in the retracted, stressed position and the extended, unstressed position, and lock the further needles 114 in the desired position. The further needles 114 may be moved between one of the these positions by pulling the release button 34 radially outwardly against the spring force, causing the coupling tube 36 to be disengaged and allowing axial movement thereof.

The hypodermic needle 100 is constructed by first fitting the needle guide 52 over the needles 114. Then, during assembly (or following disassembly for cleaning/servicing), guide 52 is slid down to cover the free ends 122 of the needles 114. The needles 114 with guide 52 in place are then inserted into the tube 112a of first needle portion part 46. When guide 52 meets the shoulder 60 of the adapter 50, the needles 114 are pushed forwards through the guide 52 and into the tube 112b of second needle portion part 48. In use, the hypodermic needle 100 is first coupled to the support tube 24. The coupling tube 36 of the syringe connector 26 is then coupled to the inner supply tube 16, and is secured to the support tube 24 by the threaded collar 32. In Fig 3A, the further hollow needles 114 are shown in the retracted, stressed position, and are locked in this retracted position by the spring-loaded release button 34. The hypodermic syringe (not shown) is then coupled to the upper inlet 38 of the syringe connector 26.

The hypodermic needle assembly may then be used for injecting a treatment fluid in an operation such as the injection of alcohol into liver tissue to treat a liver cancer. This is achieved by puncturing the external skin and intermediate tissue surrounding the liver using the first hollow needle portion 112, with the further needles 114 in the retracted position. The hypodermic needle 100 is guided by a surgeon who monitors progress of the hollow needle portion 112 through the intermediate tissue and into the liver using the MRI system.

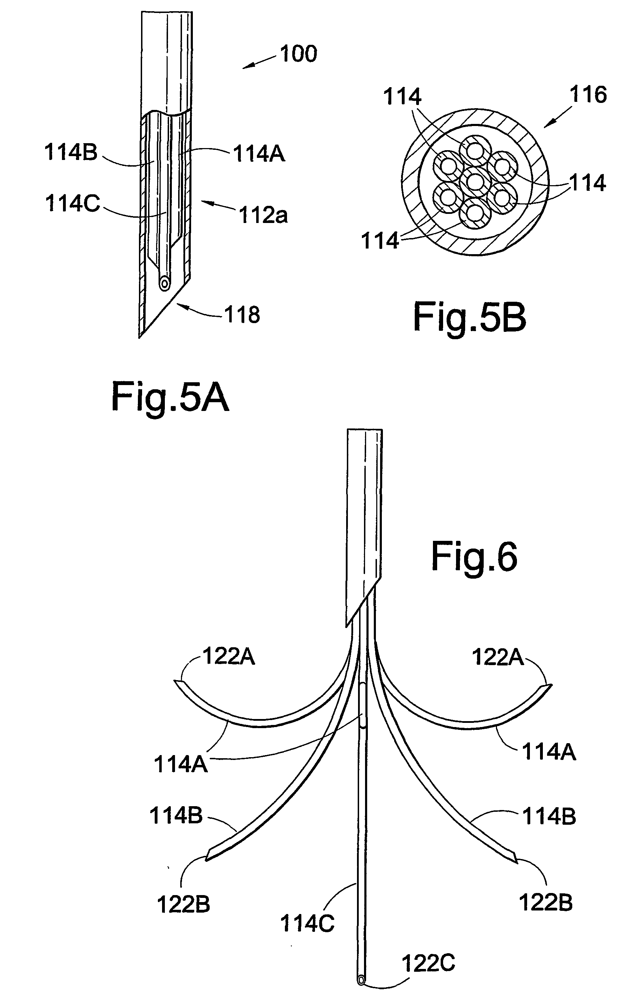

When the first needle portion 112 has penetrated the liver tissue to be treated to a sufficient depth (determined from the MRI image), the further needles 114 are moved to the extended position shown in Figs 3B and 6. This is achieved by first releasing the coupling tube 36 and inner supply tube 116 using the spring-loaded release button 34, following which the syringe connector 26 may be depressed, to move the inner supply tube 116 axially downwardly with respect to the hollow needle portion 112. This causes the further hollow needles 114 to assume their extended, unstressed positions shown in Figs 3B and 6. When the further needles 114 have been fully extended, the spring-loaded release button 34 retains the coupling tube 36, and thus the inner supply tube 116 and further needles 114, in the extended position, by engaging the groove on the coupling tube 36.

The alcohol or other treatment fluid may then be injected into the liver by depressing the hypodermic syringe plunger, moving the fluid through the coupling tube 36, inner supply tube 116 and through each of the further hollow needles 114, discharging the fluid from the needle free ends 122 at a uniform flow rate, to achieve a uniform dispersion of the fluid throughout the liver tissue to be treated.

This is typically achieved using a pump such as a Harvard PHD2000 precise high-performance delivery system or a "GENIE" (trademark) pump, both commercially available from Harvard Apparatus. This allows programmable, constant injection of treatment fluid through the hypodermic needle 100.

When the alcohol has been injected into the liver, monitored using the MRI system, the further needles 114 are retracted to the retracted, stressed position, by first releasing the coupling tube 36 using the release button 34, then moving the coupling tube 36 and inner supply tube 16 axially upwardly with respect to the hollow needle portion 112. This returns the further needles 114 to the retracted position of Fig 3A.

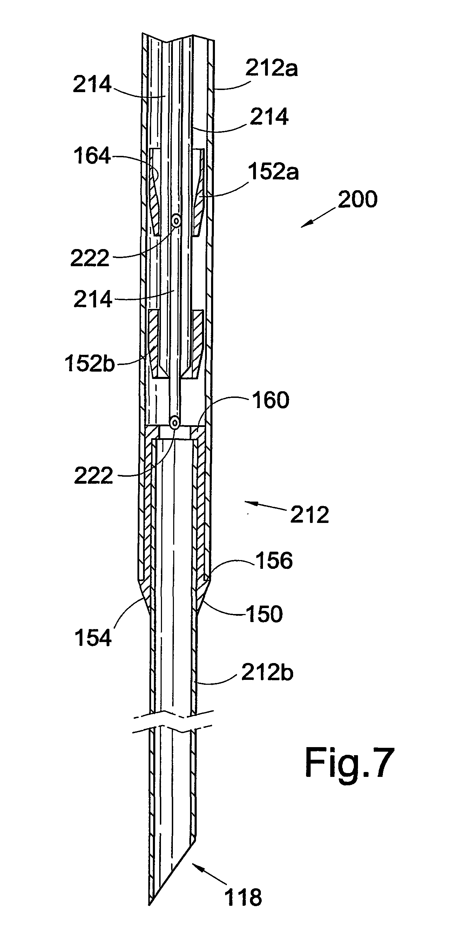

Turning now to Fig 7, there is shown part of a hypodermic needle in accordance with a further alternative embodiment of the present invention, indicated generally by reference numeral 200. The hypodermic needle 200 is similar to the needle 100 of Fig 4, and like components with the needle 100 and the needle 10, share the same reference numerals incremented by 100.

The needle 200 differs from the needle 100 of Fig 4 in that it includes a generally tubular needle guide comprising a first part 152a and a second part 152b. The first part 152a includes a tapered wall 164 which serves to move the needles to the more closely packed position. The part 152b is provided to retain the needle free ends 222 in this position. Fig 7 illustrates the further needles 214 during assembly of the hypodermic needle 200, which is carried out in a fashion similar to the needle 100.

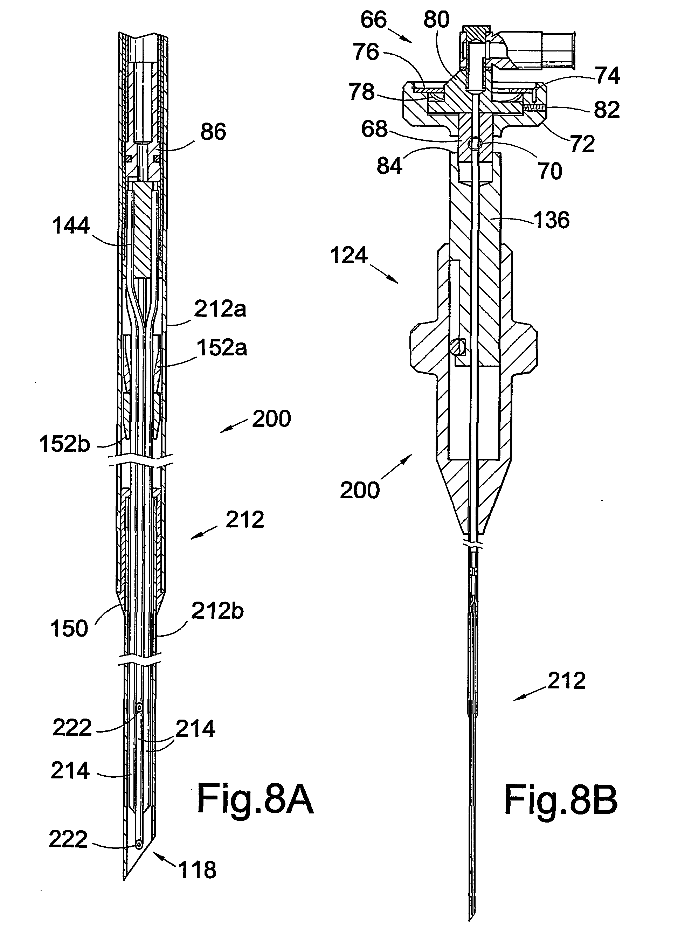

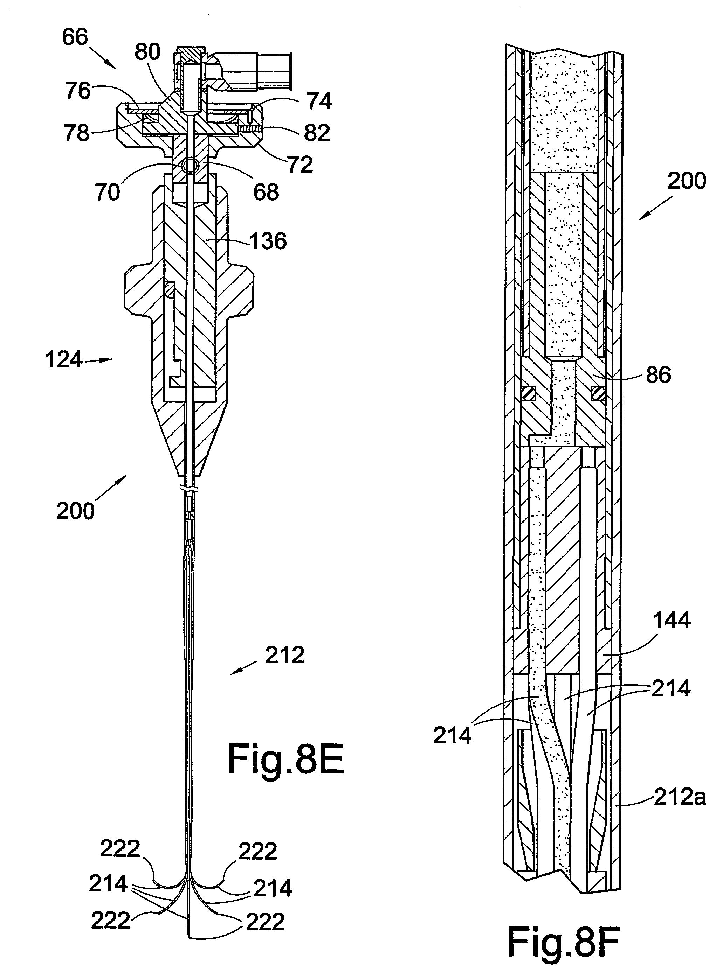

Fig 8A shows the needle 200 following insertion of the further needles 214 and with the further needles in a retracted position. Fig 8B shows the remainder of the hypodermic needle 200, which includes a support tube 214 that receives a generally tapered hollow needle portion 212 of the needle 200.

In a similar fashion to the needle 100 shown in Figs 3A and 3B, a coupling tube 136 couples the further needles 214 to a fluid supply. The coupling tube 136 includes an indexing assembly 66 for allowing selective supply through one or all of the further needles 214. Briefly, the indexing assembly 66 includes a cam boss 68 and cam lever 70 mounted in an upper end 72 of the supply tube 136. A retaining ring 74, indicator plate 76, wave spring 78, needle feed and index position switch 80 and spring plunger set screw 82 are all provided.

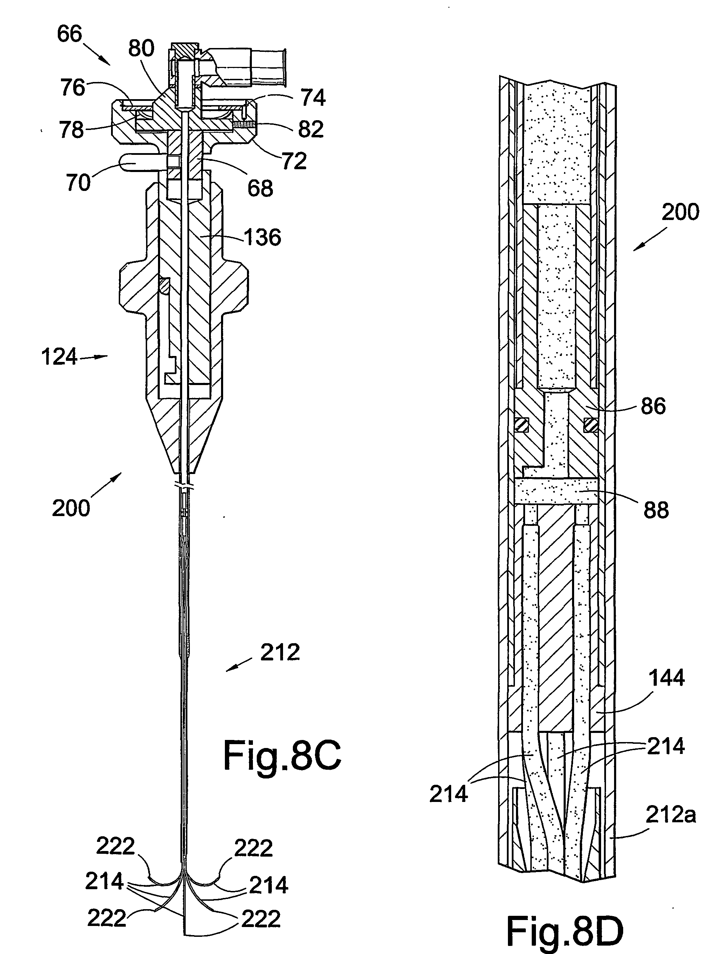

When the further needles 214 have been moved to the extended position of Fig 8C, the cam lever 70 is rotatable in a cam slot 84 between the positions of Fig 8C and Fig 8E, to allow selective supply of fluid through one or all of the further needles 214. Fig 8D shows an enlarged view of part of the needle 200 in the position of Fig 8C, and it will be seen that the needle includes a feed manifold 86. With the cam lever 70 in the position of Fig 8C, the feed manifold 86 is axially separated from needle manifold 144. This allows fluid supply to a chamber 88 and thus to all of the further needles 214.

Movement of the cam lever 70 to the position of Fig 8E both rotates and axially moves the supply tube 136 such that the feed manifold 86 is brought into sealing abutment with the needle manifold 144, to allow supply to a selected one of the needles 214, as shown.

There follows a description of the method of manufacturing the further needles 14 of the hypodermic needle 10 shown in Figs 1A to 2 and described above. It will be understood that this equally applies to the further needles 114 of the needle 100. The further needles 14, each 27 mm in length, were formed and shaped at their lower ends into a quarter circle of radius approximately 12 mm, and with a straight section of approximately 15 mm. This was achieved by placing each needle on a former, and heat treating the needles 14 at 500°C for 15 minutes, following which the needles were cold water quenched. This had the effect of "freezing" the needles 14 into the desired shape and giving them superelastic properties. SMA material in this state has been shown to have recoverable strains of up to 10% for a single strain cycle, and of 5% for greater than 1000 strain cycles. A needle 14 of inner diameter 0.42mm suffers a maximum strain of approximately 2% (approximately 100 times the wire diameter\curve diameter) when deformed uniformly from the extended position to the retracted, stressed position of Fig 1A, which is safely below the 5% multiple cycle limit. The free ends 22 of each further needle 14 were sharpened and bevelled following the heat treatment, to enable the further needles 14 to penetrate the target tissue easily.

Various modifications may be made to the foregoing within the scope of the present invention.

In a particular alternative embodiment of the present invention, the hypodermic needle 10 of Figs 1A to 2 may include a single further needle 14 which is rotatable within the first hollow outer needle 12. This may allow the further needle 14 to be rotated to inject fluid to a number of positions through a single tissue puncture of the outer needle 12. This is achieved by progressively extending the single further needle 14 (guided in an MRI procedure) before injecting, for example, treatment fluid; retracting the needle 14 and rotating it before once again extending it and injecting treatment fluid to an alternative location. This may be particularly useful for treatment of small tumours.

In the hypodermic needles 10, 100 and 200 described above, the internal diameter of the further hollow needles 14, 114 and 214 was selected to provide a relatively large resistance to flow of fluid therethrough. However, needles of greater internal diameter, with consequent lower resistance to flow may be provided as "off the shelf" components. In, in these circumstances, fluid may be injected through the further needles in short, high flow rate "pulses", to overcome any resistance to flow of the fluid in the target tissue.

Furthermore, the radius of curvature of each said further needles 14/114 may include an arc in a range of curvature of between 0 to 60° or between 110 to 180°. One or more of the further needles 14/114 may include a straight portion. The further needles 14/114 may be of a material such as steel or another non-superelastic material for needle radii of curvature outside the 60° to 110° range, and/or where an MRI guidance system is not employed. The first tapered hollow needle portion 112 may alternatively comprise a single, substantially one-piece needle which tapers to define first and second parts of different diameters, in a similar fashion to the parts 46 and 48 of the needle 100 described above.

Any suitable alternative driving means may be employed, for example, actuators for conventional hypodermic needles may be used, and the conventional hypodermic may be coupled to the needle of the invention. Such known actuators provide a constant driving force to depress the plunger of the conventional hypodermic needle.

## Claims

1. A hypodermic needle comprising a first, hollow needle having movably secured therein at least two further, hollow needles, each said further needle and part of the hypodermic needle being movable relative to one another between a stressed position of each further needle, in which each said further needle is substantially parallel to the first needle, and an unstressed position of each further needle, in which the free end of each said further needle lies beyond the axial and/or radial terminus of the first needle; and
**characterized in that** each further needle is of substantially the same length and internal diameter, for discharging a fluid from each further needle at a substantially uniform fluid flow rate, and wherein at least one of the further needles is axially staggered with respect to the at least one other further needle, such that an inlet of the at least one further staggered needle is axially spaced from an inlet of said at least one other further needle.

2. A hypodermic needle as claimed in claim 1, wherein the first, hollow needle further comprises a tapered hollow needle portion having first and second parts, the first part being of greater diameter than the second part;
wherein the hypodermic needle further comprises securing means for location in the first part of the first tapered hollow needle portion, for receiving and movably securing the at least two further hollow needles therein; and
wherein the at least two further hollow needles are more closely packed in the second part of the first hollow needle portion than in the first part.

3. A hypodermic needled as claimed in claim 2, wherein the first and second parts comprise generally tubular members, which are coupled together to form the first tapered hollow needle portion.

4. A hypodermic needle as claimed in either of claims 2 or 3, wherein the first tapered hollow needle portion further comprises a coupling for coupling the first and second needle portion parts together.

5. A hypodermic needle as claimed in any one of claims 2 to 4, wherein the first needle portion part is of an internal diameter greater than an external diameter of the second needle portion part.

6. A hypodermic needle as claimed in claim 2, wherein the first tapered hollow needle portion comprises a single, substantially one-piece needle portion, which tapers from the first diameter of the first part to the second diameter of the second part.

7. A hypodermic needle as claimed in any one of claims 2 to 6, further comprising a generally tubular needle guide adapted to be located within the first tapered hollow needle portion and tapering internally towards an end thereof disposed, in use, nearest the second needle portion part, to define a tapered passage to act as a guide for the at least two further hollow needles.

8. A hypodermic needle as claimed in claim 7, wherein the tubular needle guide comprises first and second parts which are axially separated along the further needles.

9. A hypodermic needle as claimed in any one of claims 2 to 8, wherein the securing means comprises a body for mounting the at least two further hollow needles in the first needle portion part.

10. A hypodermic needle as claimed in any preceding claim, including indexing means for allowing selective supply through one of the at least two further needles.

11. A hypodermic needle as claimed in claim 10, wherein the indexing means includes a feed manifold which is axially movable between a position where it allows supply to all of the further needles and a position where it allows supply to one or selected ones of the further needles.

12. A hypodermic needle as claimed in claim 11, wherein the feed manifold is located in the first part of the hollow needle portion and is rotatably and axially movable.

13. A hypodermic needle as claimed in any preceding claim, comprising seven further needles.

14. A hypodermic needle as claimed in preceding claim, wherein each said further needle is formed of or from a superelastic shape memory alloy.

15. A hypodermic needle as claimed in claim 14, wherein the superelastic shape memory alloy is a heat treated alloy comprising approximately 55.5% Ni and 44.5% Ti by weight.

16. A hypodermic needle as claimed in any preceding claim, wherein the first hollow needle is of a material suitable for use with a Magnetic Resonance Imaging guidance system.

17. A hypodermic needle as claimed in claim 16, wherein the first hollow needle is of a material selected from the group comprising shape memory alloys and plastics materials.

18. A hypodermic needle as claimed in any preceding claim, wherein in the unstressed position, the at least one further needle protrudes beyond both the axial and radial termini of the first needle.

19. A hypodermic needle as claimed in any preceding claim, wherein the further needles are rotatable with respect to the first needle, for locating the free end of the further needle in a desired rotational orientation with respect to the first needle.

20. A fluid injection device comprising a hypodermic needle as claimed in any one of claims 1 to 19, and driving means for driving fluid from the at least two further hollow needles at a substantially constant fluid flow rate.

21. A fluid injection device as claimed in claim 20, wherein the driving means is automatic.

## Patentansprüche

1. Hypodermische Nadel umfassend eine erste Hohlnadel, wobei diese bewegungssicher in sich wenigstens zwei weitere Hohlnadeln aufweist, wobei jede dieser weiteren Nadeln und ein Teil der hypodermischen Nadel beweglich bezüglich einander sind zwischen einer gespannten Position der jeweiligen weiteren Nadeln, wobei jede weitere Nadel im Wesentlichen parallel zu der ersten Nadel ist, und einer entspannten Position jeder weiteren Nadel, wobei das freie Ende jeder weiteren Nadel jenseits des Achsen- und/oder Radialendpunkts der ersten Nadel angeordnet ist;
**dadurch gekennzeichnet, dass**
jede weitere Nadel im Wesentlichen die gleiche Länge und den gleichen Innendurchmesser aufweist zum Absetzen eines Fluids aus jeder weiteren Nadel mit einer im Wesentlichen gleichförmigen Flussrate, wobei wenigstens eine der weiteren Nadeln axial verschoben ist in Bezug auf die wenigstens eine andere weitere Nadel, so dass ein Einlass der mindestens einen weiteren, verschobenen Nadel axial beabstandet von einem Einlass der wenigstens einen anderen weiteren Nadel liegt.

2. Hypodermische Nadel gemäß Anspruch 1, wobei die erste Hohlnadel weiterhin einen sich verjüngenden Hohlnadelabschnitt umfasst mit ersten und zweiten Teilen, wobei der erste Teil einen größeren Durchmesser aufweist als der zweite Teil;
wobei die hypodermische Nadel weiterhin ein Sicherungsmittel zur Lokalisierung im ersten Teil des ersten sich verjüngenden Hohlnadelabschnitts umfasst zum Aufnehmen und bewegbaren Sichern von den wenigstens zwei weiteren Hohlnadeln darin;
wobei die wenigstens zwei weiteren Hohlnadeln im zweiten Teil des ersten Hohlnadelabschnitts dichter aneinander angeordnet sind als im ersten Teil.

3. Hypodermische Nadel gemäß Anspruch 2, wobei die ersten und zweiten Teile im Allgemeinen rohrförmige Glieder umfassen, die aneinander gekoppelt sind, um den ersten sich verjüngenden Hohlnadelabschnitt zu bilden.

4. Hypodermische Nadel gemäß einem der Ansprüche 2 oder 3, wobei der erste sich verjüngende Hohlnadelabschnitt des weiteren eine Koppelung zum Koppeln der ersten und zweiten Nadelabschnittteile miteinander umfasst.

5. Hypodermische Nadel gemäß einem der Ansprüche 2 bis 4, wobei der erste Nadelabschnittteil einen Innendurchmesser aufweist, der größer ist als der Außendurchmesser des zweiten Nadelabschnittteils.

6. Hypodermische Nadel gemäß Anspruch 2, wobei der erste sich verjüngende Hohlnadelabschnitt einen einzelnen im Wesentlichen einteilig ausgebildeten Nadelabschnitt umfasst, der sich vom ersten Durchmesser des ersten Teils bis zum zweiten Durchmesser des zweiten Teil verjüngt.

7. Hypodermische Nadel gemäß einem der Ansprüche 2 bis 6, wobei diese weiterhin eine im Allgemeinen rohrförmige Nadelführung umfasst, die zum Anordnen innerhalb des ersten sich verjüngenden Hohlnadelabschnitts ausgebildet ist und sich innen in Richtung eines Endes derselben verjüngt und beim Gebrauch dem zweiten Nadelabschnittteil am nächsten angeordnet ist, um einen sich verjüngenden Verlauf zu definieren und als Führung für die wenigstens zwei weiteren Hohlnadeln zu dienen.

8. Hypodermische Nadel gemäß Anspruch 7, wobei die rohrförmige Nadelführung erste und zweite Teile umfasst, die axial voneinander entlang der weiteren Nadeln getrennt sind.

9. Hypodermische Nadel gemäß einem der Ansprüche 2 bis 8, wobei das Sicherungsmittel einen Körper zum Anbringen der wenigstens zwei weiteren Hohlnadeln im ersten Nadelabschnittteil umfasst.

10. Hypodermische Nadel gemäß einem der vorhergehenden Ansprüche, umfassend ein Indexierungsmittel, um eine selektive Zufuhr durch eine von den mindestens zwei weiteren Nadeln zu gestatten.

11. Hypodermische Nadel gemäß Anspruch 10, wobei das Indexierungsmittel eine Zufuhrverteilleitung enthält, die axial beweglich ist zwischen einer Position, welche die Zufuhr an alle weiteren Nadeln gestattet und einer Position, welche die Zufuhr an eine oder ausgewählte der weiteren Nadeln gestattet.

12. Hypodermische Nadel gemäß Anspruch 11, wobei die Zufuhrverteilleitung an einem ersten Teil des Hohlnadelabschnitts angeordnet sowie drehbar und axial beweglich ist.

13. Hypodermische Nadel gemäß einem der vorhergehenden Ansprüche, die sieben weitere Nadeln umfasst.

14. Hypodermische Nadel gemäß einem der vorhergehenden Ansprüche, wobei jede weitere Nadel aus oder von einer hochelastischen Legierung mit Formgedächtnis gebildet wird.

15. Hypodermische Nadel gemäß Anspruch 14, wobei die hochelastische Legierung mit Formgedächtnis eine hitzebehandelte Legierung ist, die ungefähr 55,5 % Ni und 44,5 % Ti nach Gewicht umfasst.

16. Hypodermische Nadel gemäß einem der vorhergehenden Ansprüche, wobei die erste Hohlnadel aus einem Material ist, das geeignet zum Gebrauch mit einem bildgebenden Kernspintomographie-Führungssystem ist.

17. Hypodermische Nadel gemäß Anspruch 16, wobei die erste Hohlnadel aus einem Material besteht, das aus einer Gruppe ausgewählt ist, die Legierungen mit Formgedächtnis und Kunststoffe umfasst.

18. Hypodermische Nadel gemäß einem der vorhergehenden Ansprüche, wobei in der entspannten Position die wenigstens eine weitere Nadel über die Achsen- und Radialendpunkte der ersten Nadel herausragt.

19. Hypodermische Nadel gemäß einem der vorhergehenden Ansprüche, wobei die weiteren Nadeln drehbar bezüglich der ersten Nadel sind, zum Anordnen des freien Endes der weiteren Nadel in eine gewünschte Drehausrichtung bezüglich der ersten Nadel.

20. Fluidinjektionsvorrichtung umfassend eine hypodermische Nadel gemäß einem der Ansprüche 1 bis 19 und Antriebsmittel zum Treiben von Fluid von den wenigstens zwei weiteren Hohlnadeln mit einer im wesentlichen konstanten Flussrate.

21. Fluidinjektionsvorrichtung gemäß Anspruch 20, wobei die Antriebsmittel automatisiert sind.

## Revendications

1. Aiguille hypodermique comprenant une première aiguille creuse étant fixée de manière mobile dans celle-ci, au moins deux aiguilles creuses supplémentaires, chaque dite aiguille supplémentaire et une partie de l'aiguille hypodermique pouvant se déplacer l'une par rapport à l'autre entre une position contrainte de chaque aiguille supplémentaire, dans laquelle chaque dite aiguille supplémentaire est sensiblement parallèle à la première aiguille, et une position non contrainte de chaque aiguille supplémentaire, dans laquelle l'extrémité libre de chaque dite aiguille supplémentaire se situe au-delà du bout axial et/ou radial de la première aiguille ; et
**caractérisée en ce que** chaque aiguille supplémentaire a sensiblement la même longueur et le même diamètre intérieur pour l'évacuation d'un fluide à partir de chaque aiguille supplémentaire à un débit de fluide sensiblement uniforme, et dans laquelle au moins une des aiguilles supplémentaires est décalée radialement par rapport à la au moins une autre aiguille supplémentaire, de sorte qu'un orifice d'entrée de la au moins une autre aiguille supplémentaire décalée est axialement espacé à partir d'un orifice d'entrée de la au moins une autre aiguille supplémentaire.

2. Aiguille hypodermique selon la revendication 1, dans laquelle la première aiguille creuse comprend en outre une portion d'aiguille creuse conique ayant des première et seconde parties, la première partie ayant un diamètre supérieur de la seconde partie ;
dans laquelle l'aiguille hypodermique comprend en outre des moyens de fixation pour un emplacement dans la première partie de la première portion d'aiguille creuse conique, pour recevoir et fixer de manière mobile les au moins deux aiguilles creuses supplémentaires dans celle-ci ; et
dans laquelle les au moins deux aiguilles creuses supplémentaires sont plus serrées dans la seconde partie de la première portion d'aiguille creuse que dans la première partie.

3. Aiguille hypodermique selon la revendication 2, dans laquelle les première et seconde parties comprennent généralement des éléments tubulaires, qui sont raccordés ensemble pour former la première portion d'aiguille creuse conique.

4. Aiguille hypodermique selon l'une quelconque des revendications 2 ou 3, dans laquelle la première portion d'aiguille creuse conique comprend en outre un raccord pour raccorder les première et seconde portions d'aiguille ensemble.

5. Aiguille hypodermique selon l'une quelconque des revendications 2 à 4, dans laquelle la première partie de portion d'aiguille a un diamètre intérieur supérieur à un diamètre extérieur de la seconde partie de portion d'aiguille.

6. Aiguille hypodermique selon la revendication 2, dans laquelle la première portion d'aiguille creuse conique comprend une unique portion d'aiguille sensiblement d'une seule pièce, qui diminue progressivement à partir du premier diamètre de la première partie vers le second diamètre de la seconde partie.

7. Aiguille hypodermique selon l'une quelconque des revendications 2 à 6, comprenant en outre un guide d'aiguille généralement tubulaire adapté pour être situé à l'intérieur de la première portion d'aiguille creuse conique et diminuant progressivement intérieurement vers une extrémité de celle-ci disposée, pendant l'utilisation, au plus près de la seconde partie de portion d'aiguille, pour définir un passage conique agissant comme un guide pour les au moins deux aiguilles creuses supplémentaires.

8. Aiguille hypodermique selon la revendication 7, dans laquelle le guide d'aiguille tubulaire comprend des première et seconde parties qui sont axialement séparées le long des aiguilles supplémentaires.

9. Aiguille hypodermique selon l'une quelconque des revendications 2 à 8, dans laquelle les moyens de fixation comprennent un corps pour monter les au moins deux aiguilles creuses supplémentaires dans la première partie de portion d'aiguille.

10. Aiguille hypodermique selon l'une quelconque des revendications précédentes, comprenant des moyens de repérage pour permettre une alimentation sélective au moyen de l'une des au moins deux aiguilles supplémentaires.

11. Aiguille hypodermique selon la revendication 10, dans laquelle les moyens de repérage comprennent un collecteur d'alimentation qui peut se déplacer axialement entre une position dans laquelle il permet l'alimentation de toutes les aiguilles supplémentaires et une position dans laquelle il permet l'alimentation d'une ou de plusieurs aiguilles choisies des aiguilles supplémentaires.

12. Aiguille hypodermique selon la revendication 11, dans laquelle le collecteur d'alimentation est situé dans la première partie de la portion d'aiguille creuse et peut se déplacer en rotation et axialement.

13. Aiguille hypodermique selon l'une quelconque des -revendications précédentes, comprenant sept aiguilles supplémentaires.

14. Aiguille hypodermique selon l'une quelconque des revendications précédentes, dans laquelle chaque aiguille supplémentaire est formée de ou à partir d'un alliage à mémoire de forme superélastique.

15. Aiguille hypodermique selon la revendication 14, dans laquelle l'alliage à mémoire de forme superélastique est un alliage traité thermiquement comprenant approximativement 55,8% de poids de Ni et 44,5% de poids de Ti.

16. Aiguille hypodermique selon l'une quelconque des revendications précédentes, dans laquelle la première aiguille creuse est faite dans un matériau convenant pour une utilisation dans un système de guidage d'Imagerie par Résonance Magnétique.

17. Aiguille hypodermique selon la revendication 16, dans laquelle la première aiguille creuse est faite dans un matériau choisi à partir du groupe comprenant des matériaux d'alliages à mémoire de forme et de plastiques.

18. Aiguille hypodermique selon l'une quelconque des revendications précédentes, dans laquelle en position non contrainte, la au moins une aiguille supplémentaire fait saillie au-delà à la fois des bouts axial et radial de la première aiguille.

19. Aiguille hypodermique selon l'une quelconque des revendications précédentes, dans laquelle les aiguilles supplémentaires peuvent tourner par rapport à la première aiguille, pour placer l'extrémité libre de l'aiguille supplémentaire dans une orientation tournante désirée par rapport à la première aiguille.

20. Dispositif d'injection de fluide comprenant une aiguille hypodermique selon l'une quelconque des revendications 1 à 19, et des moyens d'entraînement pour entraîner du fluide à partir des au moins deux aiguilles creuses supplémentaires à un débit de fluide sensiblement constant.

21. Dispositif d'injection de fluide selon la revendication 20, dans lequel les moyens d'entraînement sont automatiques.
